Europäisches Patentamt

European Patent Office

Office européen des brevets

(11) Publication number: **0 151 337 B1**

# EUROPEAN PATENT SPECIFICATION

(45) Date of publication of patent specification: 20.03.91

(21) Application number: 84306851.1

(22) Date of filing: 08.10.84

(51) Int. Cl.⁵: **C12N 15/00**, C12N 9/70, C12N 1/20, C07H 21/04, //(C12N1/20,C12R1:19), (C12N9/70,C12R1:19)

(54) Streptokinase-coding recombinant vectors.

(30) Priority: 10.10.83 DD 255523
02.03.84 US 585417

(43) Date of publication of application:
14.08.85 Bulletin 85/33

(45) Publication of the grant of the patent:
20.03.91 Bulletin 91/12

(84) Designated Contracting States:
AT BE CH DE FR GB IT LI NL SE

(56) References cited:
EP-A- 0 037 687
EP-A- 0 077 664
EP-A- 0 150 424
GB-A- 967 628
GB-A- 2 091 268

BIOCHEMISTRY, vol. 21, 1982, pages 6620-6625, American Chemical Society, US; K.W. JACKSON et al.: "Complete amino acid sequence of streptokinase and its homology with serine proteases"

PROC. NATL. ACAD. SCI. USA, vol. 81, June 1984, pages 3557-3561; H. MALKE et al.: "Streptokinase: cloning, expression, and excretion by Escherichia coli"

(73) Proprietor: THE BOARD OF REGENTS OF THE UNIVERSITY OF OKLAHOMA
660 Parrington Oval
Norman, Oklahoma 73019-0390(US)

(72) Inventor: Ferretti, Joseph J.
1115 Northeast 15th Street
Oklahoma City Oklahoma(US)
Inventor: Malke, Horst
Edwin-Morgner Strasse 20
O-Jena-Lobeda(DE)

(74) Representative: Baillie, Iain Cameron et al
c/o Ladas & Parry Isartorplatz 5
W-8000 München 2(DE)

## Description

### Background of the Invention

A significant medical problem, namely thromboembolism, is presented by the occlusion of blood vessels due to the presence of thrombi (blood clots). Such thromboembolisms are potentially fatal, often affecting organs such as the heart or lungs. This blockage of normal blood circulation may lead to irreversible tissue damage as metabolizing cells are deprived of oxygen, nutrients and a path for disposal of waste products. A thrombolytic event, as described above may, for example, result from the formation of a blood clot in an arteriosclerotically altered artery. Such a clot may loosen and circulate to smaller arteries until it reaches an artery or arteriole too small to permit passage of the clot. The clot thereby blocks this small artery and prevents or inhibits the passage of blood therethrough.

The restoration of normal blood flow through such clot-blocked blood vessels, particularly in time to prevent tissue damage, is an important curative goal of modern medical practice. Another medical approach to the thromboembolytic problem is to prevent the formation of vascular blood clots when conditions known to result in such clot formations are known or suspected to exist.

Certain thrombolytic agents facilitate the in vivo lysis or dissolution of blood clots. One of these agents is the bacterial protein streptokinase.

Streptokinase, a protein produced and secreted by hemolytic streptococci, was discovered and shown to cause lysis of clots containing human fibrinogen by Tillett and Garner (J. Exp. Med. 58 : 485 [1933]). While the mechanism of such fibrinolysis is not totally understood, streptokinase itself is believed to bind and activate human plasminogen, the fibrinolytically inactive precursor of plasmin. The activation of such plasminogen results in the appearance of plasmin, a fibrinolytic enzyme which hydrolyzes clot fibrinogen (Castellino TIBS, p. 1 [Jan. 1979]).

Streptokinase is a thrombolytic agent currently being used for the lysis of intravascular thrombi in pulmonary or peripheral blood vessels (Marder, Ann. Intern. Med. 90 : 802 [1979]). Recent studies of intracoronary administration of streptokinase in the treatment of acute myocardial infarction patients indicate a beneficial effect on the early course of acute myocardial infarction (Khaja et al. N.E. J. Ned. 308 , p. 1305 [1983] and Anderson et al. N.E. J. Med. 308 , p. 1312 [1983]).

Streptokinase from serological group C Streptococci has been purified for clinical usage and studied by several investigators. The primary source of this streptokinase has been the culture fluid resulting from the growth of the beta-hemolytic group C organism Streptococcus equisimilis . This culture fluid contains, in addition to streptokinase (a single-chain protein with a molecular weight of 45,000 to 50,000), a number of other extracellularly secreted substances. Since many of these other substances are toxic or potentially toxic to humans, the preparation of streptokinase from culture fluid for clinical usage involves multiple steps which must be performed with great care to ensure a clinically acceptable product.

In 1982, Jackson and Tang published what they believed to be the amino acid sequence for streptokinase (Biochemistry 21 6620-6625). Their results, however, differ from what the present inventions have found. A discussion of the differences may be found in Gene 34 357-362 (1985).

The c-DNA sequences of mammalian urokinase and tissue-type plasminogen activator have been cloned previously (Pennica et al., Nature 30 1, p. 214 [1983] and Ratzkin et al., Proc. Nat. Acad. Sci. 78 , p. 3313 [1981]).

A publication by Sako et al. (Mol. Gen. Genet. 190 , p. 271 [1983]) describes the cloning of a staphylokinase-producing Escherichia coli transformant. A polydeoxynucleotide sequence sak coding for staphylokinase, which is a plasminogen activating protein having a size of about 15 kd, was excised from a native staphylococcal-residing bacteriophage (P ø -2) and ligatively inserted into plasmid pBR322. This sak -modified plasmid was used to transform Escherichia coli K12 and the resultant staphylokinase-producing Escherichia coli transformant was found to secrete amounts of staphylokinase into a culture medium.

### Summary of the Invention

From a first aspect of the present invention provides a recombinant vector adapted for transformation of a suitable host to produce streptokinase, said recombinant vector comprising a vector into which a polydeoxyribonucleotide fragment which codes for the synthesis and secretion of streptokinase has been inserted. The transformant microorganism including this recombinant vector synthesizes and secretes streptokinase. The transformant microorganism may be cultured in a suitable nutrient medium to produce a streptokinase-rich culture, from which streptokinase may be isolated.

From another aspect the invention provides a process for preparing such a recombinant vector comprising:

digesting a vector with restriction endonuclease to produce linear vector DNA; and

ligating the linear vector DNA with a polydeoxyribonucleotide fragment which codes for synthesis and secretion of streptokinase, to produce a recombinant vector comprising a vector into which a polydeoxyribonucleotide fragment which codes for the synthesis and secretion of streptokinase has been inserted.

In a further aspect the invention provides a transformant microorganism which includes a recombinant vector of the type described above and a process of producing such a microorganism.

Finally the invention provides polydeoxyribonucleotide fragments that code for the production and secretion of streptokinase and fragments performing both of these functions.

Brief Description of the Drawings

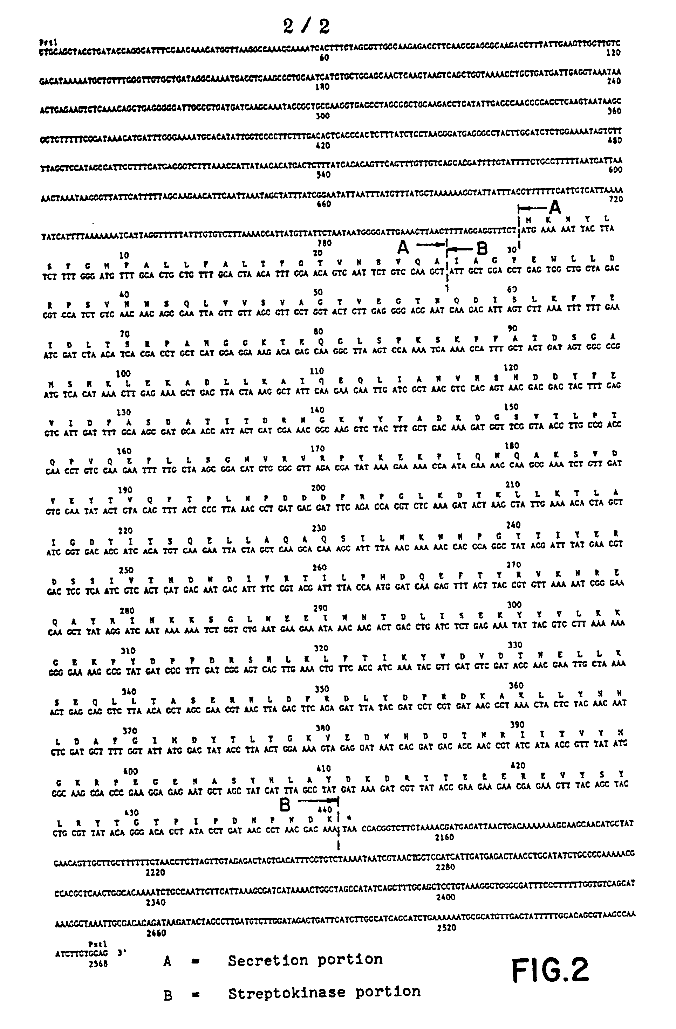

Figure I is a restriction endonuclease map of plasmid pMFI; Figure 2 illustrates the nucleotide sequence for the 2.5 kb PstI - produced pMFI fragment.

Description of the Preferred Embodiments

In accordance with the present invention, a first vector is digested with a restriction endonuclease to produce linear first vector DNA. The first vector is preferably a plasmid, which is preferably obtained from a microbial source of the genera Escherichia , Saccharomyces , Streptococci , Bacillus , Neurospora , or Streptomyces . More preferably, the first vector is a plasmid obtained from a microbial source of the species Escherichia coli . Other suitable sources for the first vector include Saccharomyces cerevisiae , Bacillus subtilus , Streptococcus sanguis , Streptococcus mutans , and Neurospora crassa . Most pre ferably, the plasmid is the plasmid pBR322, which may be obtained from Escherichia coli RRI, NRRL B-I20I4, and which has been described by Bolivar et al. (Gene. 2 , p. 95, [1977]). The restriction endonuclease for digesting the first vector is preferably either Pst I or Hind III.

The linear first vector DNA is ligated with a first polydeoxyribonucleotide fragment which codes for the synthesis and secretion of streptokinase, to produce a first recombinant vector comprising a first vector into which a polydeoxyribonucleotide fragment which codes for the synthesis and secretion of streptokinase has been inserted.

The first polydeoxyribonucleotide fragment coding for the synthesis and secretion of streptokinase is preferably obtained by digesting the chromosomal DNA of a streptokinase-producing microorganism with a restriction endonuclease, to produce at least one linear DNA fragment which codes for synthesis and secretion of streptokinase. While streptokinase appears to be exported from Streptococcus equisimilis and from pMFI-transformed Escherichia coli HBI0I, the mechanism of such extracellular export has not been completely defined. The terms 'secrete' and 'secretion,' as used in this specification and accompanying claims, are broadly meant to indicate an extracellular export of streptokinase. Preferably, the streptokinase-producing microorganism which is a source of the first polydeoxynucleotide fragment is a hemolytic microbe, and is more preferably of the genus Streptococcus , and is most preferably of the species Streptococcus equisimilis . Most preferred is Streptococcus equisimilis strain H46A, serological group C. The restriction endonuclease with which the DNA of the streptokinase-producing microorganism is digested is preferably Sau 3A or Pst I.

The polydeoxyribonucleotide fragment which codes for the synthesis and secretion of streptokinase is ligated with linear DNA from a second vector, which preferably comprises a bacteriophage, and most preferably comprises lambda L47 bacteriophage. The linear second vector DNA is preferably formed by digesting the second vector DNA with at least one restriction endonuclease, preferably Bam HI. Ligation of the linear polydeoxyribonucleotide fragment which codes for synthesis and secretion of streptokinase with linear second vector DNA results in production of a second recombinant vector. The second recombinant vector is preferably infectively added to a suitable second host, which preferably comprises a microorganism, and more preferably comprises a bacterium. The bacterium preferably is of the genus Escherichia , and is more preferably of the species Escherichia coli . The bacterium most preferably comprises Escherichia coli strain WL95 (P2). Those infected second hosts which produce streptokinase are selected and cloned.

DNA from the second recombinant vectors of streptokinase-producing second hosts is preferably partially digested with a restriction endonuclease, which preferably is the same restriction endonuclease with which the first vector is digested as described above, and which preferably comprises Hind III or Pst I. The linear polydeoxyribonucleotide fragments produced by digestion of DNA from the streptokinase-producing second recombinant vectors comprise a preferred source of first polydeoxyribonucleotide fragments which code for the synthesis and secretion of streptokinase. As described above, a first polydeoxyribonucleotide fragment coding for synthesis and secretion of streptokinase is ligated with linear first vector DNA to produce a first recombinant vector.

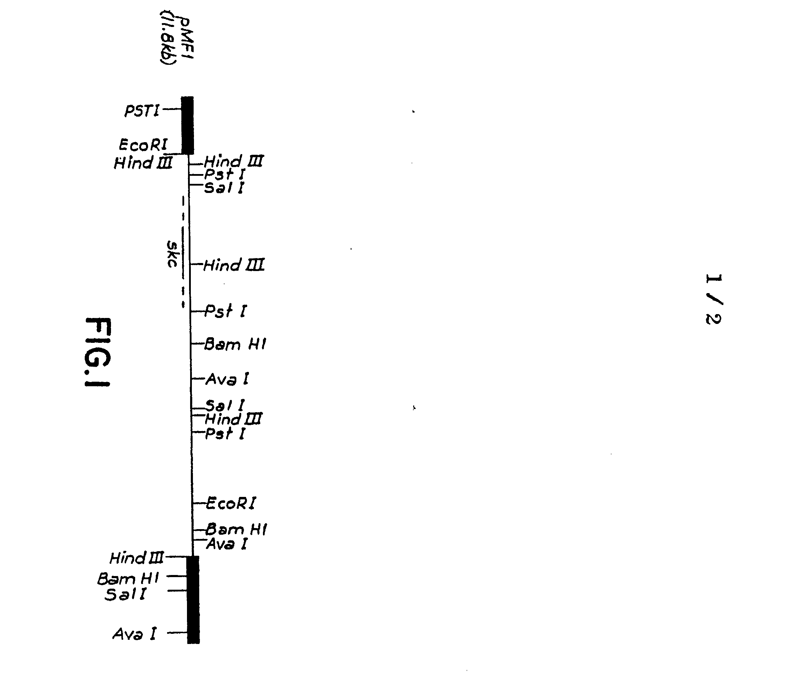

Figure 1 shows a restriction endonuclease map of the plasmid pMFI. This plasmid comprises a first recombinant vector which has been produced in accordance with the process just described, when the first vector is pBR322 and is digested by Hind III, when the streptokinase-producing microorganism is Streptococcus equisimilis strain H46A, serological group C and is digested with Sau 3A, when the second vector is lambda L47 bacteriophage and is digested with Bam HI, when the second host is Escherichia coli WL95 (P21) and when the second recombinant vector is digested with Hind III.

The first recombinant vector is infectively added to a suitable first host, which preferably comprises a microorganism, and more preferably comprises a bacterium. The bacterium is preferably of the genus Escherichia , and more preferably of the species Escherichia coli . Most preferred is the Escherichia coli strain HBIOI. This infective addition results in a streptokinase-producing transformed first host including a recombinant first vector which comprises a first vector into which a first polydeoxyribonucleotide fragment which codes for streptokinase synthesis and secretion has been inserted.

Infection of the Escherichia coli HBIOI strain with the recombinant plasmid pMFI as described above results in the transformed Escherichia coli strain HBIOI(pMFI). Cultures of Escherichia coli HBIOI(pMFI) have been deposited with the American Type Culture Collection, Rockville, Maryland, which deposit has been accorded Accession No. 3961 3, deposited February 16, 1984. The transformed first host, produced as described above, is cultured in a suitable nutrient medium to form a streptokinase-rich culture. Streptokinase thereafter may be isolated from this culture. After purification, this streptokinase is suitable for clinical fibrinolytic use.

Example I

ISOLATION OF STREPTOCOCCUS EQUISIMILIS

K46A CHROMOSOMAL DNA

Streptococcus equisimilis strain H46A (serological group C) was cultured overnight as a standing culture at 37°C. in 500 ml brain-heart infusion broth (Difco). The Streptococcus equisimilis cells were harvested by centrifugation and washed with cold TES buffer (0.03 M Tris, pH 8.0; 0.005 M EDTA; and 0.05 M NaCl). The cells were resuspended in 15 ml of 25 weight percent glucose containing 0.03 M EDTA and then mutanolysin (Dainippon Pharmaceutical Co., Osaka, Japan) dissolved in TES buffer was added to a concentration of about 2mg/ml and this cell suspension incubated at 37°C. for 30 minutes. At this point, 12.5 mg of the proteolytic enzyme Pronase, which has been predigested for 30 minutes at 37°C. as a TES solution of about 5 mg Pronase per ml, was added to the cell suspension and this resultant mixture subjected to an incubation of 37°C. for 30 minutes. After incubation the cells in the mixture were then subjected to further lysis by the addition of 10 ml of 2 weight percent sodium dodecyl sulfate in TES and 8.5 ml of 5 M sodium perchlorate in TES to produce a lysate.

The lysate was then repeatedly extracted with a phenolchloroform (2:1) solution until no further protein appeared at the aqueous-organic interface. The DNA was then precipitated from the aqueous phase by the addition of two volumes of ethanol thereto. The DNA precipitate was washed with cold 70% ethanol, dissolved in TES buffer and purified further by centrifugation in a cesium chloride-ethidium bromide buoyant density gradient. Following dialysis against 10 mM Tris, pH 7.6 with 1 mM EDTA to remove cesium chloride and ethidium bromide, the H46A DNA, having an average size of about 50kb, was stored at 40°C. -

Example II

CLONING OF STREPTOCOCCUS EQUISIMILIS H46A

DNA INTO BACTERIOPHAGE LAMBDA L47

The procedure of Cameron et al. (Nucl. Acids Res. 4 , p. 1429 [1977]) was utilized to prepare DNA from Amersham-supplied bacteriophage lambda L47 (Loenen et al., Gene 20, p. 249 [1980]) obtained from high titer bacterial lysates (i.e. containing at least $10^{10}$ PFU/ml) after precipitation of protein by sodium dodecyl sulfate-potassium sulfate additions. The bacteriophage lambda L47 KNA was digested completely with the restriction endonuclease Bam HI to form digested bacteriophage lambda L47 DNA fragments.

The Streptococcus equisimilis H46A DNA, prepared as described above in Example I, was digested with the restriction endonuclease Sau 3A according to the procedure of Maniatas et al. (Molecular Cloning: A Laboratory Manual , Cold Spring Harbor Laboratory, Cold Spring Harbor, N.Y. [1982]) to produce a mixture of H46A DNA fragments. The mixture of H46A DNA fragments was layered on a 37 ml, 10% to 40% linear sucrose

density gradient containing I mM NaCl, 20 mM Tris (pH 8.0) and 5 mM EDTA and then subjected to centrifugation at 25,000 rpm for 2I hours in a SW27 Beckman ultracentrifuge rotor. Fractions having volumes of I ml were collected from the centrifuged gradient and the size distribution of DNA fragments in each fraction was determined by electrophoresis thereof in a 0.5% agarose (Sigma) gel and comparisons of the fragment migrations with those of reference bacteriophage lambda DNA fragments having known sizes. The fractions having Streptococcus equisimilis H46A DNA fragments sized from about 4 kb to about I5 kb were pooled, dialyzed against I0 mM Tris, pH 7.4 with I mM EDTA and then precipitated with cold ethanol. The precipitated DNA fragments were dissolved in I0 mM Tris, pH 7.4 with I mM EDTA at a concentration of I mg DNA per ml.

Ligation of the Streptococcus equisimilis H46A DNA fragments with the digested bacteriophage lambda L47 DNA was accomplished by combining, in a I0 µl volume, I µg of the pooled sized H46A DNA fragments; I µg of digested lambda L47 DNA, T4 DNA ligase and ligase buffer, according to the directions supplied with the Amersham lambda DNA in vitro packaging kit. Following a I2 hour incubation at I2° C., 4 µl of the now-ligated DNA mixture was packaged into lambda phage heads according to the conditions prescribed by the Amersham instructions referred to above.

A portion of the ligated phage thus produced was infectively added to each of two Escherichia coli lawns. One lawn contained strain WL95 (P2), which allows propagation only of recombinant clones containing DNA inserts and is not susceptible to phage P2-mediated interference (Loenen et al. Gene 20, p. 249 [I980]). The other lawn contained strain WL87, which allows propagation of recombinant clones as well as of reconstituted wild type lambda L47 phage.

The total packaging efficiency calculated from the WL87 lawn was I0⁵ PFU per p.4' µg of lambda L47 DNA and these recombinant clones (obtained from the WL95 (P2) lawn) represented a library of the Streptococcus equisimilis strain H46A chromosome.

Example III

DETECTION OF RECOMBINANT PHAGES PRODUCING STREPTOKINASE

Activated plasminogen generates a caseinolytic as well as a fibrinolytic activity. The Escherichia coli which were treated with recombinant phage as described in Example II were cultured as plaques on plates of nutrient agar. These plates were then overlaid with a solution containing 50mM Tris, pH 8.I, I50 mM NaCl, I0 µg/ml human plasminogen, I0 mg agar/ml and I0 volume percent skim milk and were then incubated at 37° C. for at least 2 hours. The presence of streptokinase in a plaque resulted in the formation of a surrounding clear zone in the overlaying gelled solution, indicative of casein hydrolysis by activated plasminogen. Of the more than 8,000 plaques thus studied, I0 were found which produced surrrounding clear zones of caseinolysis. These ten recombinant streptokinase-producing clones were designated as lambda L47 (A to K) skc isolated and stored for further study.

Example IV

PHYSICAL MAPPING OF RECOMBINANT LAMBDA L47 skc CLONES

The ten recombinant streptokinase-producing lambda clones described above were isolated from high-titer lysates (I0¹⁰ PFU/ml) according to the procedures described by Davis et al. (A Manual for Genetic Engineering: Advanced Bacterial Genetics , Cold Spring Harbor Laboratory, Cold Spring Harbor, N.Y. [I980]). Restriction endonuclease digestion of the DNA from these clones was performed using the restriction endonucleases Bam HI, Eco RI and Hind III, again according to procedures described in Davis et al. The DNA digests thus obtained, together with DNA markers of known molecular sizes, were subjected to electrophoresis in I% agarose gels. The resultant gel distribution of DNA fragments from the streptokinase-producing lambda clones indicated that the size of the Streptococcus equisimilis H46A DNA fragments inserted in these clones ranged from 7 kb to I5 kb. A I.9 kb Hind III fragment was common to all ten clones which suggested that this I.9 kb fragment specified at least a part of the skc (streptokinase) gene. The patterns from lambda L47E skc and lambda L47G skc were identical.

Example V

SUBCLONING OF THE skc GENE INTO ESCHERICHIA COLI

PLASMID VECTORS

The DNA from lambda L47E skc was partially digested with the restriction endonuclease Hind III and then combined with the DNA of plasmid pBR322 which had been completely digested with the same restriction endonuclease. A ligation mixture comprising the above described digested DNA fragments, T4 DNA ligase and ligase buffer was incubated at 12° C. for 12 hours to ligate the digested plasmid pBR322 DNA with the partially digested DNA from lambda L47E skc . The ligation products were used to transform Escherichia coli strain HB101 (a commonly used strain K-12 sub-species) by following the procedure described by Dagert et al. (Gene 6 , p. 23, [1979]), to produce the transformed microorganism Escherichia coli HB101-(pMFl).

The preparation of transformed Escherichia coli strain HB101 cells was plated on LB agar plates (Lennox, Virology 1 , p. 190 [1955]) which contained 50 μg ampicillin/ml and 260 ampicillin restraint (Apr) clones were obtained. When these Apr clones were tested for growth on LB agar plates containing 12.5 μg tetracycline/ml, 37 Tcs colonies were found. When the Tcs colonies were tested for caseinolytic activity (as described in Example III) one colony was found to yield the surrounding clear zone characteristic of streptokinase production.

The plasmid obtained from this streptokinase-producing transformant was isolated by buoyant density gradient ultracentrifugation and designated plasmid pMFl. Six restriction endonucleases (Hind III, Pst I, Sal I, Bam HI, Ava I and Exo Ri) were utilized to digest plasmid pMFl by the digestion procedure described in Example IV. With the data resulting from these digestions a map of the restriction sites was determined and is shown in Figure I. Plasmid pMFl was found to have a molecular size of about 11.8 kb and contained a 7.4 kb insert in its Hind III site which included the skc gene. Partial Hind III digests of the original lambda L47E skc DNA also contained 7.4 kb fragments. Plasmid pMFl and DNA in the lambda L47E skc clone both also contained four Hind III fragments of size 2.65, 2.60, 1.9 and 0.2 kb. These identical fragments confirmed that the skc -related sequences of the lambda L47E skc DNA and the plasmid pal DNA were identical, and that the skc -related sequences of plasmid pMFl were derived from the skc -related sequences of lambda L47E.

## Example VI

## CONFIRMATION THAT THE CLONED skc GENE ORIGINATED

## IN STREPTOCOCCUS EQUISIMILIS STRAIN H46A

To conform that the cloned skc gene was that of Streptococcus equisimilis strain H46A, the hybridization procedure described by Southern (J. Mol. Biol. 98, p. 503 [1975]) was utilized.

Plasmid pMFl having a polydeoxynucleotide insert coding for streptokinase and a size of about 7.4 kb was subjected to digestion with the restriction endonuclease Pst I and the four resultant pMFl fragments having sizes of 6.0, 2.5, 2.2 and 1.1 kb were separated by electrophoresis in 0.8% low temperature melting agarose. The 2.5 kb Pst I-produced pMFl fragment was separated from the other fragments and removed from the agarose by electroelution. This purified 2.5 kb fragment was precipitated from solution by the addition of ethanol and then dissolved in 10 mM Tris, pH 7.4 containing 1 mM EDTA. The dissolved fragment was nick translated according to the procedure described by Maniatas et al. (Molecular Cloning: A Laboratory Manual , Cold Spring Harbor Laboratory, Cold Spring Harbor, N.Y. [1982]) to form a 32p-labelled 2.5 kb fragment probe. Chromosomal DNA from Streptococcus equisimilis H46A and from Escherichia coli were immobilized on separate nitrocellulose filters. The $^{32}$p-labelled fragment probe was radioautographically found to hybridize only with immobilized DNA from Streptococcus equisimilis strain H46A, thus confirming that the 2.5 kb Pst I-produced pMFl fragment originated in the Streptococcus equisimilis strain H46A.

## Example VII

## IDENTIFICATION OF THE skc GENE PRODUCT

The caseinolysis reactivity used throughout the above described procedures to monitor the skc gene product was dependent on the presence of human plasminogen, indicating that the proteolytic skc gene product was dependent upon the human plasminogen and that no independent proteolytic activity was involved. This skc -related caseinolysis reactivity did not occur when bovine plasminogen was substituted for human plasminogen in the caseinolytic assay overlay. Streptococcal streptokinase is inactive for bovine plasminogen activation.

When diisopropylfluorophosphate, an irreversible inhibitor of serine-containing proteases, was included in the caseinolytic reaction system, there was not inhibition of caseinolysis. Streptokinase, not a serine-containing protease, is similarly unaffected by diisopropylfluorophosphate.

In an Ouchterlony immunodiffusion system, antibody specific to streptokinase was placed in a center well and purified streptokinase and supernatant from a transformant Escherichia coli strain HBl0l(pMFl) culture were placed in separate surrounding wells. After diffusion through the gel an immunoprecipitate line of identity formed, indicating that streptokinase and the immunologically active component from the Escherichia coli strain HBl0l(pMFl) culture were identical.

Example VIII

EXPORT OF THE skc GENE PRODUCT BY

ESCHERICHIA COLI TRANSFORMANTS

Supernatant culture fluids from the pMFl-containing transformant of Escherichia coli strain HBl0l had levels of streptokinase activity consistent with secretion or the streptokinase from the Escherichia coli cells. Likewise, the overlaid caseinolysis assays of transformed bacteria colonies resulted in areas of casein hydrolysis having large diameters consistent with extracellular streptokinase production.

Cultures of the pMFl-transformed Escherichia coli strain HBl0l were centrifugally separated when in the early stationary growth phase to produce a supernatant fluid and cell pellet. The pelleted cells were washed and treated by the osmotic shock procedure of Hazelbauer et al. (Cell. 16 , p. 6l7 [l979]) to obtain periplasmic fluid and protein. Cytoplasmic cell proteins were obtained by the sonification of these osmotically shocked cells. When streptokinase activity was determined it was found that the supernatant fluid contained l7%; the periplasm, 30%; and the cytoplasm, 52% of the total activity. When this experiment was repeated, but with pMFl-transformed Escherichia coli HBl0l cells which had been cultured in the stationary phase for at least 8 hours, the supernatant fluid contained the majority of the streptokinase activity, while the periplasm contained an indetectably low amount and the cytoplasm contained the residue.

With Streptococcus equisimilis H46A streptokinase appears to be a secreted protein. This may indicate, according to the signal hypothesis (Emr et al., J. Cell. Biol. [l980], 86 , p. 70l; Talmadge et al., Proc. Nat'l. Acad. Sci. [l980], 77 , p. 3369; Gray et al., J. Bacteriol. [l98l], 145 , p. 422 and Roggerkamp et al., Proc. Nat'l. Acad. Sci. [l98l], 78 , p. 4466), that a precursor comprising streptokinase with an amino-terminal signal peptide is synthesized by Streptococ cus equisimilis H46A (Michaelis et al., Ann. Rev. Microbiol. [l982], 36 , p. 435; and Jackson et al., Biochem. [l982], 2l , p. 6620). The pMFl-transformed Escherichia coli HBl0l also appears to secrete streptokinase, which may indicate a commonality of secretion in gram-positive Streptococcus equisi milis H46A and gram-negative (pMFl) Escherichia coli HBl0l. The polydeoxynucleotide fragment from Streptococcus equisi milis contained in the pMFl plasmid thus may code for streptokinase as well as for an amino-terminal signal peptide which is bound to streptokinase and which is hydrolyzed during a streptokinase secretion event.

The 2.5 kb Pstl fragment, when subcloned into plasmid pBR 322, produced biologically active streptokinase, and therefore would contain all of the skc structural gene. Figure 2 indicates the nucleotide sequence for this fragment as determined by conventional sequencing procedures, those portions believed to encode for secretion ("A") and for streptokinase ("B") are also indicated.

## Claims

1. A recombinant vector adapted for transformation of a suitable host to produce streptokinase, said recombinant vector comprising a vector into which polydeoxyribo-nucleotide fragment which codes for the synthesis and secretion of streptokinase has been inserted.

2. The recombinant vector of claim l wherein the vector is a plasmid obtained from a microbial source of the genus Escherichia .

3. A recombinant vector of claim 2 wherein the microbial source of the genus Escherichia is of the species Escherichia coli .

4. The recombinant vector of claim 3 wherein the vector is plasmid pBR322.

5. The recombinant vector of claim l wherein the polydeoxyribonucleotide fragment which codes for the synthesis and secretion of streptokinase is derived from a microorganism of the genus Streptococcus .

6. The recombinant vector of claim 5 wherein the microorganism is of the species Streptococcus equisimilis .

7. The recombinant vector of claim 6 wherein the microbe is Streptococcus equisimilis strain H46A.

8. Recombinant plasmid pMFl characterized as shown by the restriction endonuclease map in Figure l of the drawings.

9. A process for preparing a recombinant vector comprising a vector into which a polydeoxyribonucleotide fragment which codes for the synthesis and secretion of streptokinase has been inserted, comprising:

digesting a vector with restriction endonuclease to produce linear vector DNA; an

ligating the linear vector DNA with a polydeoxyribonucleotide fragment which codes for synthesis and secretion of streptokinase, to produce a recombinant vector comprising a vector into which a polydeoxyribonucleotide fragment which codes for the synthesis and secretion of streptokinase has been inserted.

10. The process of claim 9 in which the polydeoxyribonucleotide fragment is prepared by digesting chromosomal DNA of a streptokinase producing microorganism with the same restriction endonuclease used to digest the vector, to produce a polydeoxyribonucleotide fragment which codes for the synthesis and secretion of streptokinase.

11. The process of claim 9 in which the restriction endonuclease is Pst I.

12. The process of claim 10 in which the restriction endonuclease is Pst I.

13. The process of claim 9 wherein the vector is a plasmid obtained from a microbial source of the genus Escherichia .

14. The process of claim 13 wherein the microbial source of the genus Escherichia is of the species Escherichia coli .

15. The recombinant vector of claim 14 wherein the vector is plasmid pBR322.

16. The process of claim 9 wherein the polydeoxyribonucleotide fragment which codes for the synthesis and secretion of streptokinase is derived from a microorganism of the genus Streptococcus .

17. The process of claim 16 wherein the microorganism is of the species Streptococcus equisimilis .

18. The process of claim 17 wherein the microorganism is Streptococcus equisimilis strain H46A.

19. A transformant microorganism which includes a recombinant vector comprising a vector into which a polydeoxyribonucleotide fragment which codes for the synthesis and secretion of streptokinase has been inserted.

20. The transformant microorganism of claim 19 which is of the genus Escherichia .

21. The transformant microorganism of claim 20 which is of the species Escherichia coli .

22. The transformant microorganism of claim 21 which is Escherichia coli strain HBI0I.

23. The transformant microorganism of claim 19 wherein the vector is the plasmid pBR322.

24. The transformant microorganism of claim 19 wherein the recombinant vector is plasmid pMFI as shown by the restriction endonuclease map of Figure I of the drawings.

25. Escherichia coli HBI0I (pMFI) (ATCC 396I3).

26. A process for preparing a streptokinase-producing microorganism which comprises transforming into a suitable microorganism host a recombinant vector comprising a vector into which a polydeoxyribonucleotide fragment which codes for the synthesis and secretion of streptokinase has been inserted.

27. The process of claim 26 in which the microorganism host is of the genus Escherichia .

28. The process of claim 27 in which the microorganism host is of the species Escherichia coli .

29. The process of claim 28 in which the microorganism host is Escherichia coli HBI0I.

30. The process of claim 26 in which the recombinant vector is pMFI as shown by the restriction endonuclease map in Figure I of the drawings.

31. A polydeoxyribonucleotide fragment which codes for synthesis and secretion of streptokinase.

32. The polydeoxyribonucleotide fragment of claim 3I which has restriction endonuclease cleavage sites at the termini thereof.

33. The polydeoxyribonucleotide fragment of claim 32 which has about 7400 deoxyribonucleotide pairs.

34. The polydeoxyribonucleotide sequence of claim 32 in which the cleavage sites are for the restriction endonuclease Hind III.

35. The polydeoxyribonucleotide fragment of claim 3I which is derived from a microorganism of the genus Strepto coccus .

36. The polydeoxyribonucleotide fragment of claim 35 wherein the microorganism is Streptococcus equisimilis strain H46A.

37. The polydeoxyribonucleotide fragment of claim 3I having a nucleotide sequence substantially as illustrated in Figure 2.

38. The polydeoxyribonucleotide fragment of claim 3I having a nucleotide sequence substantially as illustrated in the portions of Figure 2 identified as A and B.

39. A polydeoxyribonucleotide fragment which codes for streptokinase.

40. The polydeoxyribonucleotide fragment of claim 39 having a nucleotide sequence substantially as illustrated in the portion of Figure 2 identified as B.

41. A polydeoxyribonucleotide fragment which codes for secretion of streptokinase.

42. The polydeoxyribonucleotide fragment of claim 4I having a nucleotide sequence substantially as illustrated in the portion of Figure 2 identified as A.

43. A process for producing streptokinase comprising:
   culturing a transformant microorganism, which includes a recombinant vector comprising a vector into which a polydeoxyribonucleotide fragment which codes for the synthesis and secretion of streptokinase has been inserted, in a suitable nutrient medium to form streptokinase; and
   isolating streptokinase from said culture.

44. The process of claim 37 wherein the transformant microorganism is of the species Escherichia coli .

45. The process of claim 38 in which the transformant microorganism is Escherichia coli HBIOI (pMFI). (ATCC 39I3).

**Revendications**

1. Vecteur recombinant adapté pour la transformation d'un hôte approprié pour produire de la streptokinase, ledit vecteur recombinant comprenant un vecteur dans lequel a été inséré un fragment de polydésoxyribonucléotide qui code pour la synthèse et la sécrétion de la streptokinase.

2. Vecteur recombinant de la revendication I où le vecteur est un plsamide obtenu à partir d'une source microbienne du genre Escherichia .

3. Vecteur recombinant de la revendication 2 dans lequel la source microbienne du genre Escherichia est de l'espèce Escherichia coli .

4. Vecteur recombinant de la revendication 3 où le vecteur est le plasmide pBR322.

5. Vecteur recombinant de la revendication I dans lequel le fragment de polydésoxyribonucléotide qui code pour la synthèse et la sécrétion de la streptokinase est dérivé d'un microorganisme du genre Streptococcus .

6. Vecteur recombinant de la revendication 5 où le microorganisme est de l'espèce Streptococcus equisimilis .

7. Vecteur recombinant de la revendication 6 où le microbe est Streptococcus equisimilis souche H46A.

8. Plasmide recombinant pMFI caractérisé comme le montre la carte d'endonucléase de restriction de la figure I des dessins.

9. Procédé de préparation d'un vecteur recombinant comprenant un vecteur dans lequel un fragment de polydésoxyribonucléotide qui code pour la synthèse et la sécrétion de la streptokinase a été inséré, dans lequel:
   on digère un vecteur avec une endonucléase de restriction pour produire un ADN de vecteur linéaire; et
   on lie l'ADN de vecteur linéaire avec un fragment de polydésoxyribonucléotide qui code pour la synthèse et la sécrétion de la streptokinase, pour produire un vecteur recombinant comprenant un vecteur dans lequel a été inséré un fragment de polydésoxyribonucléotide qui code pour la synthèse et la sécrétion de la streptokinase.

10. Procédé de la revendication 9 dans lequel on prépare le fragment de polydésoxyribonucléotide en digérant l'ADN chromosomique d'un microorganisme producteur de streptokinase avec la même endonucléase de restriction que celle utilisée pour digérer le vecteur, afin de produire un fragment de polydésoxyribonucléotide qui code pour la synthèse et la sécrétion de la streptokinase.

11. Procédé de la revendication 9 dans lequel l'endonucléase de restriction est Pst I.

12. Procédé de la revendication l0 dans lequel l'endonucléase de restriction est Pst I.

13. Procédé de la revendication 9 dans lequel le vecteur est un plasmide obtenu à partir d'une source microbienne du genre Escherichia .

14. Procédé de la revendication l3 dans lequel la souce microbienne du genre Escherichia est de l'espèce Escherichia coli .

15. Vecteur recombinant de la revendication l4 où le vecteur est le plasmide pBR322.

16. Procédé de la revendication 9 dans lequel le fragment de polydésoxyribonucléotide qui code pour la synthèse et la sécrétion de la streptokinase est dérivé d'un microorganisme du genre Streptococcus .

17. Procédé de la revendication l6 dans lequel le microorganisme est de l'espèce Streptococcus equisimilis .

18. Procédé de la revendication l7 dans lequel le microorganisme est Streptococcus equisimilis souche H46A.

19. Microorganisme transformant qui inclut un vecteur recombinant, comprenant un vecteur dans lequel a été inséré un fragment de polydésoxyribonucléotide qui code pour la synthèse et la sécrétion de la streptokinase.

20. Microorganisme transformant de la revendication l9 qui est du genre Escherichia .

21. Microorganisme transformant de la revendication 20 qui est de l'espèce Escherichia coli .

22. Microorganisme transformant de la revendication 2l qui est Escherichia coli souche HBl0l.

23. Microorganisme transformant de la revendication l9 dans lequel le vecteur est le plasmide pBR322.

24. Microorganisme transformant de la revendication l9 dans lequel le vecteur recombinant est le plasmide pMFl comme le montre la carte d'endonucléase de restriction de la figure l des dessins.

25. Escherichia coli HBl0l (pMFl) (ATCC 396l3).

26. Procédé de préparation d'un microorganisme producteur de streptokinase dans lequel on transforme dans un microorganisme hôte approprié un vecteur recombinant comprenant un vecteur dans lequel a été inséré un fragment de polydésoxyribonucléotide qui code pour la synthèse et la sécrétion de la streptokinase.

27. Procédé de la revendication 26 dans lequel le microorganisme hôte est du genre Escherichia .

28. Procédé de la revendication 27 dans lequel le microorganisme hôte est de l'espèce Escherichia coli .

29. Procédé de la revendication 28 dans lequel le microorganisme hôte est Escherichia coli HBl0l.

30. Procédé de la revendication 26 dans lequel le vecteur recombinant est pMFl comme le montre la carte d'endonucléase de restriction dans la figure l des dessins.

31. Fragment de polydésoxyribonucléotide qui code pour la synthèse et la sécrétion de la streptokinase.

32. Fragment de polydésoxyribonucléotide de la revendication 3l qui présente des sites de clivage d'endonucléase de restriction à ses extrémités.

33. Fragment de polydésoxyribonucléotide de la revendication 32 qui a environ 7400 paires de désoxyribonucléotides.

34. Séquence de polydésoxyribonucléotide de la revendication 32 dans laquelle les sites de clivage sont pour l'endonucléase de restriction Hind III.

35. Fragment de polydésoxyribonucléotide de la revendication 3l qui est dérivé d'un microorganisme du genre Streptococcus .

36. Fragment de polydésoxyribonucléotide de la revendication 35 dans lequel le microorganisme est Streptococcus equisimilis souche H46A.

37. Fragment de polydésoxyribonucléotide de la revendication 3l ayant une séquence nucléotidique essentiellement telle que présentée dans la figure 2.

38. Fragment de polydésoxyribonucléotide de la revendication 3l ayant une séquence nucléotidique essentiellement telle que présentée dans les parties de la figure 2 identifiées par A et B.

39. Fragment de polydésoxyribonucléotide qui code pour la streptokinase.

40. Fragment de polydésoxyribonucléotide de la revendication 39 ayant une séquence nucléotidique essentiellement telle que présentée dans la partie de la figure 2 identifiée par B.

41. Fragment de polydésoxyribonucléotide qui code pour la sécrétion de la streptokinase.

42. Fragment de polydésoxyribonucléotide de la revendication 4l ayant une séquence nucléotidique essentiellement telle que présentée dans la partie de la figure 2 identifiée par A.

43. Procédé de production de streptokinase dans lequel: on cultive un microorganisme transformant, qui inclut un vecteur recombinant comprenant un vecteur dans lequel a été inséré un fragment de polydésoxyribonucléotide qui code pour la synthèse et la sécrétion de la streptokinase, dans un milieu nutritif approprié pour former la streptokinase; et on isole la streptokinase à partir de ladite culture.

44. Procédé de la revendication 37 dans lequel le microorganisme transformant est de l'espèce Escherichia coli .

45. Procédé de la revendication 38 dans lequel le microorganisme transformant est Escherichia coli HBl0l (pMFl) (ATCC 396l3).

## Ansprüche

1. Rekombinanter Vektor, der zur Transformation eines geeigneten Wirtes unter Bildung von Streptokinase geeignet ist, welcher rekombinante Vektor einen Vektor umfaßt, in den ein Polydesoxyribonucleotidfragment insertiert wurde, das für die Synthese und Sekretion von Streptokinase codiert.

2. Rekombinanter Vektor nach Anspruch l, wobei der Vektor ein Plasmid ist, das von einer Mikrobenquelle der Gattung Escherichia erhalten wurde.

3. Rekombinanter Vektor nach Anspruch 2, wobei die Mikrobenquelle der Gattung Escherichia von der Art Escherichia coli ist.

4. Rekombinanter Vektor nach Anspruch 3, wobei der Vektor Plasmid pBR322 ist.

5. Rekombinanter Vektor nach Anspruch l, wobei das Polydesoxyribonucleotidfragment, das für die Synthese und Sekretion von Streptokinase codiert, von einem Mikroorganismus der Gattung Streptococcus stammt.

6. Rekombinanter Vektor nach Anspruch 5, wobei der Mikroorganismus von der Art Streptococcus equisimilis ist.

7. Rekombinanter Vektor nach Anspruch 6, wobei die Mikrobe Streptococcus equisimilis Stamm H46A ist.

8. Rekombinantes Plasmid pMFl, charakterisiert wie durch die Restriktionsendonucleasekarte in Fig. l der Zeichnungen gezeigt.

9. Verfahren zum Herstellen eines rekombinanten Vektors umfassend einen Vektor, in den ein Polydesoxyribonucleotidfragment insertiert wurde, das für die Synthese und Sekretion von Streptokinase codiert, umfassend:
   Digerieren eines Vektors mit Restriktionsendonuclease zur Bildung von linearer Vektor-DNA;
   Verknüpfen der linearen Vektor-DNA mit einem Polydesoxyribonucleotidfragment, das für die Synthese und Sekretion von Streptokinase codiert, zur Bildung eines rekombinanten Vektors, der einen Vektor umfaßt, in den ein Polydesoxyribonucleotidfragment insertiert wurde, das für die Synthese und Sekretion von Streptokinase codiert.

10. Verfahren nach Anspruch 9, in dem das Polydesoxyribonucleotidfragment durch Digerieren von chromosomaler DNA eines streptokinaseproduzierenden Mikroorganismus mit der gleichen Restriktionsendonuclease, die zum Digerieren des Vektors verwendet wurde, zur Bildung eines Polydesoxyribonucleotidfragments, das für die Synthese und Sekretion von Streptokinase codiert, hergestellt wird.

11. Verfahren nach Anspruch 9, in dem die Restriktionsendonuclease Pst l ist.

12. Verfahren nach Anspruch l0, in dem die Restriktionsendonuclease Pst I ist.

13. Verfahren nach Anspruch 9, in dem der Vektor ein Plasmid erhalten von einer Mikrobenquelle der Gattung Escherichia ist.

14. Verfahren nach Anspruch l3, in dem die Mikrobenquelle der Gattung Escherichia von der Art Escherichia coli ist.

15. Verfahren nach Anspruch l4, in dem der Vektor Plasmid pBR322 ist.

16. Verfahren nach Anspruch 9, in dem das Polydesoxyribonucleotidfragment, das für die Synthese und Sekretion von Streptokinase codiert, von einem Mikroorganismus der Gattung Streptococcus stammt.

17. Verfahren nach Anspruch l6, in dem der Mikroorganismus von der Art Streptococcus equisimilis ist.

18. Verfahren nach Anspruch l7, in dem der Mikroorganismus Streptococcus equisimilis Stamm H46A ist.

19. Transformand-Mikroorganismus, der einen rekombinanten Vektor umfassend einen Vektor, in den ein Polydesoxyribonucleotidfragment insertiert wurde, das für die Synthese und Sekretion von Streptokinase codiert, einschließt.

20. Transformand-Mikroorganismus nach Anspruch l9, der von der Gattung Escherichia ist.

21. Transformand-Mikroorganismus nach Anspruch 20, der von der Art Escherichia coli ist.

22. Transformand-Mikroorganismus nach Anspruch 2l, der Escherichia coli Stamm HBlOl ist.

23. Transformand-Mikroorganismus nach Anspruch l9, in dem der Vektor das Plamid pBR322 ist.

24. Transformand-Mikroorganismus nach Anspruch l9, in dem der rekombinante Vektor Plasmid pMFl ist, wie durch die Restriktionsendonucleasekarte der Fig. l der Zeichnungen gezeigt.

25. Escherichia coli HBlOl (pMFl) (ATCC 396l3).

26. Verfahren zum Herstellen eines streptokinaseproduzierenden Mikroorganismus, das das Transformieren eines rekombinanten Vektors umfassend einen Vektor, in den ein Polydesoxyribonucleotidfragment insertiert wurde, das für die Synthese und Sekretion von Streptokinase codiert, in einen geeigneten Mikroorganismenwirt umfaßt.

27. Verfahren nach Anspruch 26, in dem der Mikroorganismenwirt von der Gattung Escherichia ist.

28. Verfahren nach Anspruch 27, in dem der Mikroorganismenwirt von der Art Escherichia coli ist.

29. Verfahren nach Anspruch 28, in dem der Mikroorganismenwirt Escherichia coli HBlOl ist.

30. Verfahren nach Anspruch 26, in dem der rekombinante Vektor pMFl ist, wie durch die Restriktionsendonucleasekarte in Fig. l der Zeichnungen gezeigt.

31. Polydesoxyribonucleotidfragment, das für die Synthese und Sekretion von Streptokinase codiert.

32. Polydesoxyribonucleotidfragment nach Anspruch 3l, das Restriktionsendonucleaseschnittstellen an den Termini aufweist.

33. Polydesoxyribonucleotidfragment nach Anspruch 32, das etwa 7400 Desoxyribonucleotidpaare aufweist.

34. Polydesoxyribonucleotidfragment nach Anspruch 32, in der die Schnittstellen für die Restriktionsendonuclease Hind III sind.

35. Polydesoxyribonucleotidfragment nach Anspruch 31, das von einem Mikroorganismus der Gattung Streptococcus stammt.

36. Polydesoxyribonucleotidfragment nach Anspruch 35, in dem der Mikroorganismus Streptococcus equisimilis Stamm H46A ist.

37. Polydesoxyribonucleotidfragment nach Anspruch 3l mit einer Nucleotidsequenz, im wesentlichen wie in Fig. 2 illustriert.

38. Polydesoxyribonucleotidfragment nach Anspruch 3l mit einer Nucleotidsequenz, im wesentlichen wie in den als A und B identifizierten Teilen der Fig. 2 illustriert.

39. Polydesoxyribonucleotidfragment, das für Streptokinase codiert.

40. Polydesoxyribonucleotidfragment nach Anspruch 39 mit einer Nucleotidsequenz, im wesentlichen wie in dem als B identifizierten Teil der Fig. 2 illustriert.

41. Polydesoxyribonucleotidfragment, das für die Sekretion von Streptokinase codiert.

42. Polydesoxyribonucleotidfragment nach Anspruch 4I mit einer Nucleotidsequenz, im wesentlichen wie in dem als A identifizierten Teil der Fig. 2 illustriert.

43. Verfahren zum Herstellen von Streptokinase, das das Züchten eines Transformand-Mikroorganismus, der einen rekombinanten Vektor umfassend einen Vektor, in den ein Polydesoxyribonucleotidfragment insertiert wurde, das für die Synthese und Sekretion von Streptokinase codiert, einschließt, in einem geeigneten Nährmedium zum Bilden von Streptokinase und das Isolieren von Streptokinase von der Kultur umfaßt.

44. Verfahren nach Anspruch 37, in dem der Transformand-Mikroorganismus von der Art Escherichia coli ist.

45. Verfahren nach Anspruch 38, in dem der Transformand-Mikroorganismus Escherichia coli HBIOI(pMFI) (ATCC 39613) ist.

FIG.1

## 2 / 2

```
PstI
CTGCAGCTACCTGATACCAGGCATTTCCAACAAACATGGTTAAGGCCAAACCAAAATCACTTTCTAGCGTTGGCAAGAGACCTTCAAGCCGAGCGCAAGACCTTTATTGAAGTTGCTTGTC
                                                      60                                                              120

GACATAAAAATGCTGTTTGGGTTGTGCTGATAGGCAAAATGACCTCAAGCCCTGCAATCATCTGCTGGAGCAACTCAACTAAGTCAGCTGGTAAAACCTGCTGATGATTGAGGTAAATAA
                                                      180                                                             240

ACTGAGAAGTCTCAAACAGCTGAGGGGGATTGCCCTGATGATCAAGCAAATACCGCTGCCAAGGTGACCCTAGCGGCTGCAAGACCTCATATTGACCCAACCCCACCTCAAGTAATAAGC
                                                      300                                                             360

GCTCTTTTTCGGATAAACATGATTTGGGAAAATGCACATATTGGTCCCCTTCTTTGACACTCACCCACTCTTTATCTCCTAACGGATGAGGGCCTACTTGCATCTCTGGAAAATAGTCTT
                                                      420                                                             480

TTAGCTCCATAGCCATTCCTTTCATGACGGTCTTTAAACCATTATAACACATGACTCTTTATCACACAGTTCAGTTTGTTGTCAGCACGATTTTGTATTTTCTGCCTTTTTAATCATTAA
                                                      540                                                             600

AACTAAATAAGGCGTTATTCATTTTTAGCAAGAACATTCAATTAAATAGCTATTTATCGGAATATTAATTTATGTTTATGCTAAAAAAGGTATTATTTACCTTTTTTCATTGTCATTAAAA
                                                      660                                                             720
```

```
                                                                                                        ┌──► A
                                                                                                    M  K  N  Y  L
TATCATTTTAAAAAAATCATTAGGTTTTTATTTGTGTGTCTTTAAAACCATTATGTTATTCTAATAATGGGGATTGAAACTTAACTTTTAGGCAGGTTTCT│ATG AAA AAT TAC TTA
                                       780                                      A ──►│  ┌──► B
                                                      20                                    30
```

```
          10                                                              A ──►│  ┌──► B  30
  S  F  G  M  F  A  L  L  F  A  L  T  F  G  T  V  N  S  V  Q  A│ I  A  G  P  E  W  L  L  D
TCT TTT GGG ATG TTT GCA CTG CTG TTT GCA CTA ACA TTT GGA ACA GTC AAT TCT GTC CAA GCT│ATT GCT GGA CCT GAG TGG CTG CTA GAC

          40                                          50                                          60
  R  F  S  V  N  N  S  Q  L  V  V  S  V  A  G  T  V  E  G  T  N  Q  D  I  S  L  K  F  F  E
CGT CCA TCT GTC AAC AAC AGC CAA TTA GTT GTT AGC GTT CCT GGT ACT CTT GAG GGG ACG AAT CAA GAC ATT AGT CTT AAA TTT TTT GAA

          70                                          80                                          90
  I  D  L  T  S  R  P  A  N  G  G  K  T  E  Q  G  L  S  P  K  S  K  P  F  A  T  D  S  G  A
ATC GAT CTA ACA TCA CGA CCT GCT CAT GGA GGA AAG ACA GAG CAA GGC TTA AGT CCA AAA TCA AAA CCA TTT GCT ACT GAT AGT GGC CCG

          100                                         110                                         120
  M  S  N  K  L  E  K  A  D  L  L  K  A  I  Q  E  Q  L  I  A  N  V  H  S  N  D  D  Y  F  E
ATG TCA CAT AAA CTT GAG AAA GCT GAC TTA CTA AAG GCT ATT CAA GAA CAA TTG ATC GCT AAC GTC CAC AGT AAC GAC GAC TAC TTT GAG

          130                                         140                                         150
  V  I  D  F  A  S  D  A  T  I  T  D  R  N  G  K  V  Y  F  A  D  K  D  G  S  V  T  L  P  T
GTC ATT GAT TTT GCA AGC GAT GCA ACC ATT ACT GAT CGA AAC GGC AAG GTC TAC TTT GCT GAC AAA GAT GGT TCG GTA ACC TTG CCG ACC

          160                                         170                                         180
  Q  P  V  Q  E  F  L  L  S  G  H  V  R  V  R  P  Y  K  E  K  P  I  Q  N  Q  A  K  S  V  D
CAA CCT GTC CAA GAA TTT TTG CTA AGC GGA CAT GTG CGC GTT AGA CCA TAT AAA GAA AAA CCA ATA CAA AAC CAA GCG AAA TCT GTT GAT

          190                                         200                                         210
  V  E  Y  T  V  Q  F  T  P  L  N  P  D  D  D  F  R  P  G  L  K  D  T  K  L  L  K  T  L  A
GTG GAA TAT ACT GTA CAG TTT ACT CCC TTA AAC CCT GAT GAC GAT TTC AGA CCA GGT CTC AAA GAT ACT AAG CTA TTG AAA ACA CTA GCT

          220                                         230                                         240
  I  G  D  T  I  T  S  Q  E  L  L  A  Q  A  Q  S  I  L  N  K  N  H  P  G  Y  T  I  Y  E  R
ATC GGT GAC ACC ATC ACA TCT CAA GAA TTA CTA GCT CAA GCA CAA AGC ATT TTA AAC AAA AAC CAC CCA GGC TAT ACG ATT TAT GAA CGT

          250                                         260                                         270
  D  S  S  I  V  T  H  D  N  D  I  F  R  T  I  L  P  H  D  Q  E  F  T  Y  R  V  K  N  R  E
GAC TCC TCA ATC GTC ACT CAT GAC AAT GAC ATT TTC CGT ACG ATT TTA CCA ATG GAT CAA GAG TTT ACT TAC CGT GTT AAA AAT CGG GAA

          280                                         290                                         300
  Q  A  Y  R  I  N  K  K  S  G  L  N  E  E  I  N  N  T  D  L  I  S  E  K  Y  Y  V  L  K  K
CAA GCT TAT AGG ATC AAT AAA AAA TCT GGT CTG AAT GAA GAA ATA AAC AAC ACT GAC CTG ATC TCT GAG AAA TAT TAC GTC CTT AAA AAA

          310                                         320                                         330
  G  E  K  P  Y  D  P  F  D  R  S  H  L  K  L  F  T  I  K  Y  V  D  V  D  T  N  E  L  L  K
GGG GAA AAG CCG TAT GAT CCC TTT GAT CGC AGT CAC TTG AAA CTG TTC ACC ATC AAA TAC GTT GAT GTC GAT ACC AAC GAA TTG CTA AAA

          340                                         350                                         360
  S  E  Q  L  L  T  A  S  E  R  N  L  D  F  R  D  L  Y  D  P  R  D  K  A  K  L  L  Y  N  N
ACT GAG CAG CTC TTA ACA GCT AGC GAA CGT AAC TTA GAC TTC AGA GAT TTA TAC GAT CCT CGT GAT AAG GCT AAA CTA CTC TAC AAC AAT

          370                                         380                                         390
  L  D  A  F  G  I  M  D  Y  T  L  T  G  K  V  E  D  N  H  D  D  T  N  R  I  I  T  V  Y  M
CTC GAT GCT TTT GGT ATT ATG GAC TAT ACC TTA ACT GGA AAA GTA GAG GAT AAT CAC GAT GAC ACC AAC CGT ATC ATA ACC GTT TAT ATG

          400                                         410                                         420
  G  K  R  P  E  G  E  N  A  S  Y  H  L  A  Y  D  K  D  R  Y  T  E  E  E  R  E  V  Y  S  Y
GGC AAG CGA CCC GAA GGA GAG AAT GCT AGC TAT CAT TTA GCC TAT GAT AAA GAT CGT TAT ACC GAA GAA GAA CGA GAA GTT TAC AGC TAC

          430              B ──►│  440
  L  R  Y  T  G  T  P  I  P  D  N  P  N  D  K│◄
CTG CGT TAT ACA GGG ACA CCT ATA CCT GAT AAC CCT AAC GAC AAA│TAA CCACGGTCTTCTAAAACGATGAGATTAACTGACAAAAAAAGCAAGCAACATGCTAT
                                                             2160
```

```
CAACAGTTGCTTGCTTTTTTCTAACCTCTTAGTTGTAGAGACTAGTGACATTTCGTGTCTAAAATAATCGTAACTGGTCCATCATTGATGAGACTAACCTGCATATCTGCCCCAAAAACG
2220                                                                                    2280

CCACGCTCAACTGGCACAAAATCTGCCCAATTGTTCATTAAAGCCGATCATAAAACTGGCTAGCCATATCAGCTTTGCAGCTCCTGTAAAGGCTGGGCGATTTCCCTTTTTTGGTGTCAGCAT
2340                                                                                    2400

AAAGCGTAAATTGCCGACACAGATAAGATACTACCCTTGATGTGTCTTGGATAGACTGATTCATCTTGCCATCAGCATCTGAAAAAATGCGGCATGTTGACTATTTTTGCACAGCCGTAAGCCAA
2460                                                                                    2520
```

```
PstI
ATCTTCTGCAG 3'
2568
```

A = Secretion portion

B = Streptokinase portion

# FIG.2